# EUROPEAN PATENT SPECIFICATION

(11) **EP 3 644 899 B1**
(45) Date of publication and mention of the grant of the patent: **09.08.2023**
(21) Application number: 18823549.3
(22) Date of filing: 29.06.2018
(51) Int. Cl.: A61F 2/38, A61F 2/46

(54) **PATELLA KIT**
PATELLA-KIT
KIT DE PATELLA

(30) Priority: 30.06.2017 US 201762527547 P
(43) Date of publication of application: 06.05.2020
(73) Proprietor: Exactech, Inc., Gainesville, FL 32653 (US)
(72) Inventor: ANGIBAUD, Laurent, Gainesville, FL 32653 (US); STROUD, Nick, Gainesville, FL (US); LANSDOWN, Jason, Gainesville, FL 32653 (US)
(74) Representative: Meissner Bolte Partnerschaft mbB
(86) International application number: PCT/US2018/040404
(87) International publication number: WO 2019/006370

(56) References cited:
- US-A1- 2007 239 165
- US-A1- 2009 264 894
- US-A1- 2011 054 486
- US-A1- 2012 109 150
- US-A1- 2013 079 671
- US-A1- 2013 310 977
- US-A1- 2017 156 798

## Description

### Field of the Invention

The field of invention relates to surgical repair of the knee joint. More particularly, the field of invention relates to total knee arthroplasty ("TKA"), and most notably to the patellofemoral joint of the TKA.

### Background of the Invention

TKA has been considered a safe and effective treatment for end-stage knee arthritis. More than 90% of people who have had total knee replacement surgery experience a dramatic reduction of knee pain and a significant improvement in the ability to perform common activities of daily living. Within the first five years after primary implantation, the revision rate is 2.8%, and the rate of reoperations without exchange of components is 4.3%.

Though TKA is a very successful procedure, approximately 5-10% of the patients experience anterior knee pain after TKA, with reports ranging from 0.4% to 49%. After joint infection, patellofemoral problems are a reason for re-operation or revision following TKA. Because of the required management of pain and risk of re-operation, the patellofemoral problems represent a substantial additional health care cost.

The causes of anterior knee pain are multifactorial and can be divided into functional (e.g., muscle imbalances, dynamic valgus) and mechanical (e.g., poor alignment and incorrect positioning of prosthetic components) problems. The mechanical causes of patellofemoral problems after knee replacement can be distinguished according to whether they increase instability in the joint, lead to changes in the transmission of the joint forces and contact pressures, or whether they affect the muscular lever arms. These contributing factors are patellofemoral instability, prosthesis design, patella baja/alta, chondrolysis, anteroposterior offset error of the femoral component, internal-external rotation of the femoral component, tibiofemoral instability, avascular necrosis and synovial hyperplasia. All of these causes can lead to pain and functional deficit of patients with anterior knee pain after TKA.

US 2009/264894 A1 relates to orthopedic implants and methods of designing orthopedic implants using in vivo data specific to an orthopedic implant or orthopedic trial.

US 2013/079671 A1 relates to an insert sensing device for measuring a parameter of the muscular-skeletal system. The insert sensing device can be temporary or permanent.

US 2011/054486 A1 relates to methods of selecting and implanting prosthetic devices for use as a replacement meniscus.

### Summary of the Invention

The invention is defined in claim 1. Exemplary embodiments relate to a system for use in joint repair surgery.

In an embodiment (not part of the invention), a computer-implemented method includes positioning at least one sensor within a joint of a patient, wherein the sensor is configured to record at least one reaction force within the joint while the joint is moved through a range of motion; receiving, by a processor, from the at least one sensor positioned within the joint of the patient, a first data set representing a first series of reaction forces within the joint while the joint is moved through a range of motion during a first time period; receiving, by the processor, from the at least one sensor positioned within the joint of the patient, a second data set representing a second series of reaction forces within the joint while the joint is moved through the range of motion during a second time period, wherein a first trial implant is attached within the joint during the second time period; calculating, by the processor, a difference between the second data set and the first data set; and providing, by the processor, an instruction based on the difference, wherein the instruction includes either (a) an instruction to select a second trial implant if the difference exceeds a threshold, or (b) an instruction to proceed with an actual implant matching the first trial implant if the difference does not exceed the threshold.

In an embodiment (not part of the invention), the step of calculating the difference between the second data set and the first data set includes determining whether the difference between the second data set and the first data set is statistically significant. In an embodiment, a least squares method is used to determine whether the difference between the second data set and the first data set is statistically significant.

In an embodiment, the threshold is either an absolute threshold or a relative threshold. In an embodiment, the difference between the second data set and the first data set includes a different orientation, and the second trial implant has a different angular offset than the first trial implant, and wherein the at least one sensor comprises a plurality of sensors.

In an embodiment, the at least one sensor includes at least one of a resistance element, a capacitance element, a piezo-resistance element, a piezoelectric element, a hydraulic element, a pneumatic element, a magneto-elastic element, a vibration element, an optical element, or combinations thereof. In an embodiment, the difference between the second data set and the first data set includes a different magnitude, and wherein the second trial implant has a different thickness than the first trial implant.

In an embodiment, the joint is a knee joint of a human, and the first and second trials are patella trials. In an embodiment, the difference between the first data set and the second data set includes a positional offset, and the second trial implant has a different positional offset than the first trial implant.

According to the invention, a system for use in joint repair surgery includes a sensing device configured for implantation within a joint of a patient, the sensing device including at least one sensor that is configured to record at least one reaction force within the joint while the joint is moved through a range of motion; a plurality of trial implants configured for trial implantation within the joint of the patient, each of the plurality of trial implants being configured to simulate one of a corresponding plurality of implants configured for permanent implantation within the joint; and a non-transient memory electronically storing computer executable program code, the computer executable program code configured such that a processor, when executing the program code, becomes a specifically programmed computing processor that is configured to perform at least the following operations: receiving, from the sensing device while the sensing device is positioned within a joint of a patient, a first data set representing a first series of reaction forces within the joint while the joint is moved through a range of motion during a first time period; receiving, from the sensing device while the sensing device is positioned within the joint of the patient, a second data set representing a second series of reaction forces within the joint while the joint is moved through the range of motion during a second time period, wherein a first one of the trial implants is attached within the joint during the first time period; calculating a difference between the second data set and the first data set; and providing an instruction based on the difference, wherein the instruction includes either (a) an instruction to select a second one of the trial implants if the difference exceeds a threshold, or (b) an instruction to proceed with an implant matching the first one of the trial implants if the difference does not exceed the threshold.

In an embodiment, the system also includes a plurality of actual implants, each of the plurality of actual implants corresponding to one of the plurality of trial implants. In an embodiment, the plurality of trial implants are patellar trials. In an embodiment, the threshold is either an absolute threshold or a relative threshold.

In an embodiment, the sensing device comprises a plurality of force sensors, and wherein the difference between the second data set and the first data set includes a different orientation, and wherein the second one of the trial implants has a different angular offset than the first one of the trial implants. In an embodiment, the sensing device includes at least one of a resistance element, a capacitance element, a piezo-resistance element, a piezoelectric element, a hydraulic element, a pneumatic element, a magneto-elastic element, a vibration element, an optical element, or combinations thereof.

According to the invention, the second data set and the first data set includes a different magnitude, and the second one of the trial implants has a different thickness than the first one of the trial implants, or the difference between the first data set and the second data set includes a positional offset, and the second one of the trial implants has a different positional offset than the first one of the trial implants. According to the invention, the sensing device includes a rail, and the at least one sensor is movable linearly along the rail.

In an embodiment, the operation of calculating the difference between the second data set and the first data set includes determining whether the difference between the second data set and the first data set is statistically significant. In an embodiment, a least squares method is used to determine whether the difference between the second data set and the first data set is statistically significant.

### Brief Description of the Figures

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.
Figure 1 shows a first exemplary characterization kit;
Figure 2A shows a front view of a second exemplary characterization kit;
Figure 2B shows a side view of the exemplary characterization kit of Figure 2A;
Figure 3A shows a side view of an implantation location of the exemplary characterization kit of Figure 1;
Figure 3B shows a top view of the implantation location shown in Figure 3A;
Figure 4A shows a front view of an exemplary patella prior to resection;
Figure 4B shows a side view of an exemplary patella prior to resection;
Figure 4C shows a side view of the exemplary patella of Figure 4B after resection;
Figure 5 shows an exemplary resected patella with implant sizing guides overlaid;
Figure 6 shows an exemplary resected patella with the exemplary characterization kit of Figure 2A attached thereto;
Figure 7A shows an exemplary curve of force with respect to flexion angle;
Figure 7B shows an exemplary curve of mediolateral position with respect to flexion angle corresponding to the exemplary curve of Figure 7A;
Figure 8A shows an Input data set of force with respect to flexion angle;
Figure 8B shows a first Output data set of force with respect to flexion angle for comparison to the Input data set of Figure 8A;
Figure 8C shows a second Output data set of force with respect to flexion angle for comparison to the Input data set of Figure 8A;
Figure 9A shows an Input data set of force with respect to flexion angle;
Figure 9B shows a first Output data set of force with respect to flexion angle for comparison to the Input data set of Figure 9A;
Figure 9C shows a second Output data set of force with respect to flexion angle for comparison to the Input data set of Figure 9A;
Figure 10A shows an Input data set of mediolateral position with respect to flexion angle;
Figure 10B shows a first Output data set of mediolateral position with respect to flexion angle for comparison to the Input data set of Figure 10A;
Figure 10C shows a second Output data set of mediolateral position with respect to flexion angle for comparison to the Input data set of Figure 10A;
Figure 11 shows an exemplary resected native patella with holes drilled therein for implantation of a patella implant;
Figure 12 shows a flowchart of an exemplary method that may be practiced by an exemplary computing device;
Figure 13A shows an Input data set of interior-exterior rotation with respect to flexion angle;
Figure 13B shows a first Output data set of interior-exterior rotation with respect to flexion angle for comparison to the Input data set of Figure 13A;
Figure 13C shows a second Output data set of interior-exterior rotation with respect to flexion angle for comparison to the Input data set of Figure 13A;
Figure 14 schematically illustrates the elements of an exemplary inventive computing device;
Figure 15 shows a third exemplary characterization kit as affixed to a resected patella;
Figure 16A shows a photograph of an exemplary prototype of a sensor;
Figure 16B shows an anatomical model of a knee joint instrumented with the sensor shown in Figure 16A to perform an initial characterization of the knee joint;
Figure 16C shows an anatomical model of a knee joint instrumented with the sensor shown in Figure 16A to perform a subsequent characterization of the knee joint;
Figure 16D shows the anatomical model of Figure 16C during the characterization;
Figure 17A shows a 3D envelope representing data recorded during a first characterization;
Figure 17B shows a 3D envelope representing data recorded during a second characterization as compared to the 3D envelope shown in Figure 17A;
Figure 17C shows the provision of a recommended replacement trial based on the comparison shown in Figure 17B;
Figure 17D shows a 3D envelope representing data recorded during a third characterization using the recommended replacement trial shown in Figure 17C, as compared to the 3D envelope shown in Figure 17A;
Figure 18A shows a 3D envelope representing data recorded during a first characterization;
Figure 18B shows a 3D envelope representing data recorded during a second characterization as compared to the 3D envelope shown in Figure 18A;
Figure 18C shows the provision of a recommended replacement trial based on the comparison shown in Figure 18B;
Figure 18D shows a 3D envelope representing data recorded during a third characterization using the recommended replacement trial shown in Figure 18C, as compared to the 3D envelope shown in Figure 18A;
Figure 19A shows a 3D envelope representing data recorded during a first characterization;
Figure 19B shows a 3D envelope representing data recorded during a second characterization as compared to the 3D envelope shown in Figure 19A;
Figure 19C shows the provision of a recommended replacement trial based on the comparison shown in Figure 19B;
Figure 19D shows a 3D envelope representing data recorded during a third characterization using the recommended replacement trial shown in Figure 19C, as compared to the 3D envelope shown in Figure 19A;
Figure 20A shows an exemplary patellar component and femoral component with well-aligned contact;
Figure 20B shows the exemplary patellar component and femoral component of Figure 20A with misaligned contact;
Figure 20C shows the exemplary femoral component of Figure 20A with an exemplary angulated patellar component; and
Figure 20D shows well-aligned contact between the exemplary patellar component and femoral component of Figure 20C.

### Detailed Description of the Invention

Among those benefits and improvements that have been disclosed, other objects and advantages of this invention will become apparent from the following description taken in conjunction with the accompanying figures. Detailed embodiments of the present invention are disclosed herein; however, it is to be understood that the disclosed embodiments are merely illustrative of the invention that may be embodied in various forms. In addition, each of the examples given in connection with the various embodiments of the invention which are intended to be illustrative, and not restrictive.

Throughout the specification and claims, the following terms take the meanings explicitly associated herein, unless the context clearly dictates otherwise. The phrases "in one embodiment," "in an embodiment," and "in some embodiments" as used herein do not necessarily refer to the same embodiment(s), though it may. Furthermore, the phrases "in another embodiment" and "in some other embodiments" as used herein do not necessarily refer to a different embodiment, although it may. Thus, as described below, various embodiments of the invention may be readily combined, without departing from the scope of the invention as defined by the claims.

As used herein, the term "based on" is not exclusive and allows for being based on additional factors not described, unless the context clearly dictates otherwise. In addition, throughout the specification, the meaning of "a," "an," and "the" include plural references. The meaning of "in" includes "in" and "on."

The exemplary embodiments relate to TKA with or without resurfacing of the patella. More particularly, the exemplary embodiments relate to a patella kit system that may reduce the occurrence of anterior knee pain by providing active guidance to the surgeon regarding the selection of the optimal patella implant determined by characterization of the patellofemoral joint in terms of load intensity, load distribution and/or tracking of said values over the range of flexion.

In some examples, a patella kit system includes a first characterization kit, a second characterization kit, a specifically programmed/configured inventive computing device and an implant kit. In some examples, the first characterization kit is configured to perform characterization of the patellofemoral joint before resurfacing of the patella. In some examples, the second characterization kit is configured to perform characterization of the patellofemoral joint after resurfacing of the patella. In some examples, the exemplary inventive computing device receives data from the first characterization kit and the second characterization kit. In some examples, based on treatment of the data, the exemplary inventive computing device may be programmed/configured to display guidance, including, without limitation, a recommendation for selecting an optimal patella implant from the patella kit and/or how to optimally position the optimal patella implant. In some examples, as explained later in this disclosure, the guidance is based on the comparison of a characterization performed at an early stage during the surgical procedure (referred to herein as "Input") and a characterization performed at a later stage with the trial components in place (referred to herein as "Output"). In some examples, in case of discrepancy between the two sets of characterization data, the exemplary inventive computing device issues guidance in order to decrease the level of discrepancy. In some examples, assuming the change has been implemented, exemplary inventive computing device may be programmed/configured to receive data resulting from a subsequent characterization with the modified parameters in order to confirm the improvement.

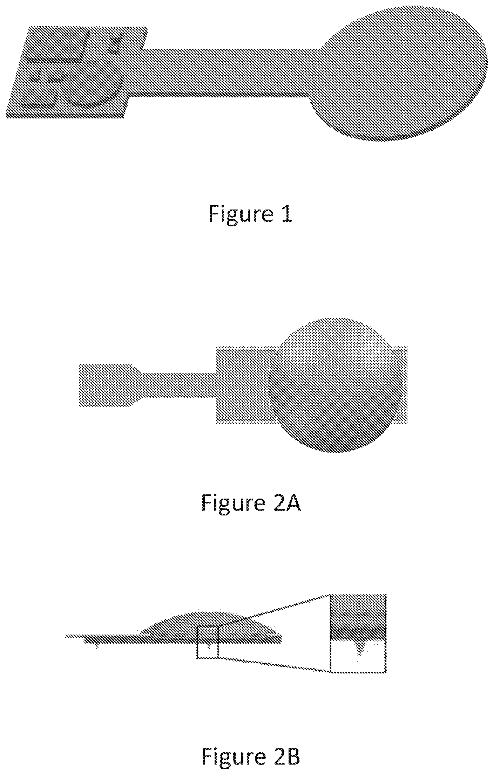

Figure 1 illustrates a first exemplary characterization kit. In some examples, the characterization kit includes a conforming sensor module and a wireless module. In some examples, the conforming sensor module is configured to be attached to the articular surface of the native patella. In some examples, the sensor module is configured to be attached by a suturing process or other known technique of attaching two components together (e.g., mechanical attachment, chemical attachment, etc.). In some examples, a single sensor module is configured for use on all sized patellae. In some examples, in order to ascertain that the conforming sensor module is only sensing where boney contact occurs, the device includes a mechanism of reducing the sensing area of the sensor module in order to reduce the incidence of noise in the sensed data. In some examples, the sensing area is reduced by physically reducing the actual sensing area to be the same as that of the native patella. In some examples, exemplary sensor module is configured to allow the sensing area to be reduced by receiving data regarding a diameter of the area to be sensed from a separate device (e.g., the exemplary inventive computing device) via the wireless module. In some examples, once the sensing area is defined, the exemplary sensing module is configured to acquire data regarding a series of joint analyses performed by a practitioner (e.g., a surgeon) after the practitioner has reduced the patella to its native position. In some examples, the wireless module communicates sensed data to the exemplary inventive computing device, which is programmed/configured to wirelessly receive data from the wireless module and provide real-time visualization relative to the contact between the conforming sensor and the femur in terms of force and moment (e.g., total force, center of force, moment in the internal-external axis, moment in the medial-lateral axis resulting) or location.

In some examples, the wireless module is physically attached to the sensing component and is configured to be positioned so as not to interfere with joint kinematics. In some examples, the wireless module includes a processing unit and a component for wireless communication. In some examples, characterization performed with the first characterization kit can be boney (for example, if performed before the preparation of the distal femur and before the preparation of the patella). In some examples, characterization performed with the first characterization kit can be hybrid (for example, if performed after the preparation of the distal femur and before the preparation of the patella).

Figure 2A shows a top view of a second exemplary characterization kit. Figure 2B shows a side view of the second exemplary characterization kit of Figure 2A. In some embodiments, a second characterization kit includes an instrumented patella trial component comprised of (i) a low-profile rail element featuring two opposite preferably planar surfaces, where a first surface is intended to and/or configured to be placed against the previously resected native patella, while the second surface features a sensor array; (ii) a plurality of mobile patella trial elements featuring a representative articular surface of different implants and a substantially planar opposite surface, where said substantially planar surface is intended to and/or configured to contact the second surface of the low-profile rail element and, in some embodiments, slide along the low-profile rail element; and (iii) a wireless module able to transmit data collected by the sensor array attached to the low-profile rail element, preferably at one of its extremities. In some embodiments, the first surface of the rail element includes at least one spike configured to securely connect the rail element to the resected surface of the native patella. In some embodiments, the sensor array comprises at least two rows of force sensitive aspects. In some embodiments, the substantially planar surface of the patella trial elements does not slide along the low-profile rail element.

In some embodiments, the sensor array includes any type of sensing element that is capable of sensing a force, pressure, and/or strain. In various embodiments, sensing elements operate through the use of resistance, capacitance, piezo-resistance, piezoelectric, hydraulic, pneumatic, magneto-elastic, vibration, optical, or any other appropriate methods. In some embodiments, the output of the sensing elements may be interpreted electrically or optically by a computing device. Thus, when this disclosure uses generic terms such as "sensor," "sensing element," "force sensor," and the like, it will be apparent to those of skill in the art that these terms may refer to any suitable type of sensor such as those described above.

In some embodiments, characterization performed with the second characterization kit can be hybrid (for example, if performed before the preparation of the distal femur and after the preparation of the patella). In some embodiments, characterization performed with the second characterization kit can be prosthetic (for example, if performed after the preparation of the distal femur and after the preparation of the patella).

In some embodiments, a computing device is configured to wirelessly receive data from the wireless modules of the first and second characterization kits. In some embodiments, the computing device is configured to wirelessly receive data from the wireless module of the first characterization kit relative to the contact between the first characterization kit and the distal femur. In some embodiments, the computing device is configured to wirelessly receive data from the wireless module of the first characterization kit relative to the contact between the first characterization kit and the femoral component trial. In some embodiments, the computing device is configured to wirelessly receive data from the wireless module of the second characterization kit relative to the contact between the second characterization kit and the distal femur. In some embodiments, the computing device is configured to wirelessly receive data from the wireless module of the second characterization kit relative to the contact between the second characterization kit and the femoral component trial. In some embodiments, the computing device is configured to receive any of the above data in terms of force and moment (e.g., total force, center of force, moment in the internal-external axis, moment in the medial-lateral axis resulting) or location. In some embodiments, the computing device is configured to treat the data from the characterization(s) and provide real time information and guidance to the surgeon through a display system or other known suitable media able to communicate information and guidance. In some embodiments, the computing device includes a computer, a tablet, a smartphone, or another suitable computing device. In some embodiments, the computing device includes hardware and software provided in conjunction with a patella kit system. In some embodiments, the computing device includes software provided in conjunction with a patella kit system and configured to be installed on existing computing hardware.

In some embodiments, an implant kit includes a plurality of patella implants. In some embodiments, the patella implants are monoblock patella implants. In some embodiments, the patella implants are metal-backed patella implants. In some embodiments, the patella implants are modular metal-backed patella implants. In some embodiments, each of the patella implants includes a generally dome-shaped articular surface and a generally planar opposite surface. In some embodiments, pegs extend from the planar surface of each of the patella implants and are to be anchored into the resected native patella. In some embodiments, the patella implants are available with different outer diameters, different thicknesses (defined as the maximum distance from the planar surface to the zenith of the dome-shaped articular surface), and different angulations (defined as the angle formed by the planar surface and the perimeter of the dome-shaped articular surface). In one embodiment, the table below illustrates the scope of an implant kit including four anatomical sizes, three thickness options, and two angulation sub-options, resulting in twenty-four (24) patella implants:

| Size | A | | | | | | B | | | | | | C | | | | | | D | | | | | |
|---|---|---|---|---|---|---|---|---|---|---|---|---|---|---|---|---|---|---|---|---|---|---|---|---|
| Thickness* | - | | 0 | | + | | - | | 0 | | + | | - | | 0 | | + | | - | | 0 | | + | |
| Angulation** | N | W | N | W | N | W | N | W | N | W | N | W | N | W | N | W | N | W | N | W | N | W | N | W |
| *: where "-" relates to a patella implant thinner than "0" and "+" relates to a patella implant thicker than "0" | | | | | | | | | | | | | | | | | | | | | | | | |
| **: where "N" relates to a neutral angulation and "W" relates to a wedged design | | | | | | | | | | | | | | | | | | | | | | | | |

More generally, a kit providing a quantity *m* sizes, each available under a quantity *n* thickness options, each thickness available under a quantity p angulation sub-options, would include a total quantity (m * n * p) patella implants. In some embodiments, there is a dimensional compatibility between the instrumented patella trial and the implant kit. In some embodiments, the diameter of at least one given mobile patella trial element is substantially the same as the diameter of at least one given patella implant. In some embodiments, the combined thickness of at least one given mobile patella trial element assembled with the low-profile rail element is substantially the same as the thickness of at least one given patella implant. In some embodiments, the angulation of at least one given mobile patella trial element is substantially the same as the angulation of at least one given patella implant.

In some embodiments, an optimal implant from the implant kit is selected by comparison between a selected boney or hybrid characterization (Input) and a prosthetic characterization (Output). In some embodiment, in case of noticeable discrepancy between the two sets of characterization, then the computing device issues guidance (1) to select an optimum implant from the kit and (2) to position the selected optimum implant in order to obtain substantial match between the prosthetic characterization and the initial boney or hybrid characterization.

The following discussion illustrates the sequences of the use of an exemplary embodiment of a patella kit system considering a TKA with resurfacing of the patella and where the mobile patella trial is able to freely translate along a defined axis of the low-profile rail element. (As noted above, in other embodiments, the patella trial does not translate along the low-profile rail element.) This discussion will be with reference to other figures that will be described hereinafter; it is understood that the following figures are only intended to facilitate the explanation of this embodiment and shall not be considered as limiting the breadth, scope, or applicability of the proposed embodiment. Additionally, the following sequences only focus on the steps relative to the preparation of the patella and most notably those associated with the characterizations of the patellofemoral joint and the selection of the optimum patella implant resulting from the said characterization. The steps associated with the preparation of the proximal tibia and distal femur are performed according to known techniques.

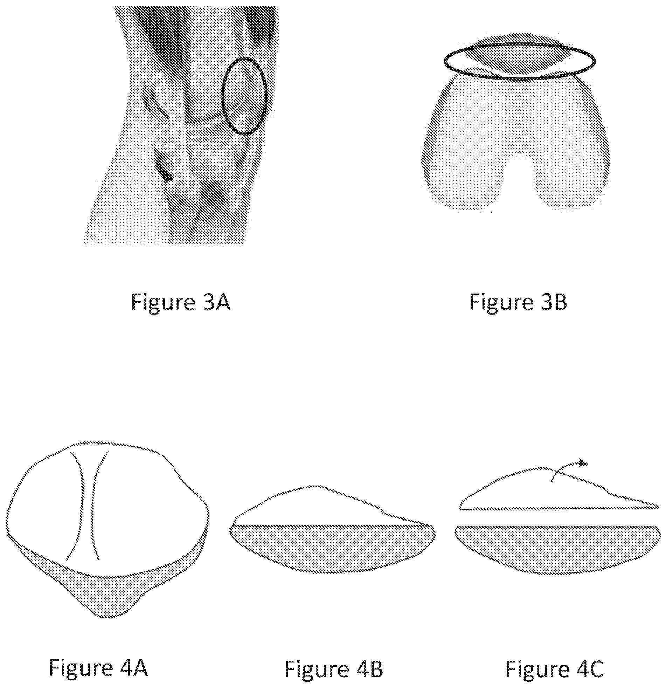

In some embodiments, prior to performing any bone cut, a boney characterization is performed by attaching the conforming sensor module of the first characterization kit to the native patella, defining the profile of the conforming sensor, reducing the patella to its native position, and performing a series of joint analyses in order to capture a boney characterization of the patellofemoral joint. Figures 3A and 3B illustrate the position of the conforming sensor module within the knee. Figure 3A shows a side view, while Figure 3B shows a top view. The position of the conforming sensor module is circled in both Figures 3A and 3B.

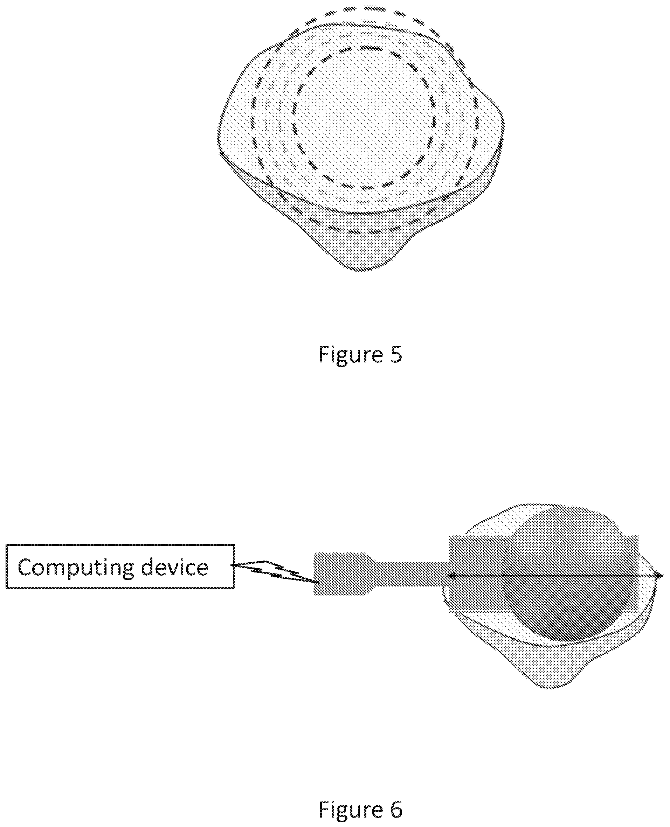

In some embodiments, the native patella is prepared in accordance with known techniques. In some embodiments, the surgeon resects the articular portion of the native patella at a given depth and orientation. Figures 4A, 4B, and 4C show the resection of the native patella. Figure 4A shows a view of the rear surface of the patella prior to resection, Figure 4B shows a side view of the patella prior to resection, and Figure 4C shows a side view of the patella after resection. It will be understood by those of skill in the art that resection parameters are subjective in nature and based on surgeons' perceptions. In addition, some surgeons may reference the amount resected, while others may reference the thickness of bone that will be left once resection is completed. In some embodiments, the surgeon defines an appropriate dimension (e.g., diameter if circular contour or proximal-distal height if non-circular contour) of the mobile patella trial element to be selected by comparing the resected surface of the native patella against templates according to known techniques. Figure 5 shows an exemplary comparison of a resected native patella to variously sized trial elements. In some embodiments, the selected mobile patella trial element is the one associated with the size offering the optimum coverage of the resected native patella, while not overhanging it. In such an embodiment, the selected mobile patella trial element would be the one sized as the second smallest concentric circle shown in Figure 5, which is the largest circle that does not overhang the edges of the native patella.

In some embodiments, once the relevant dimension is defined, the surgeon assembles the selected mobile patella trial element of the second characterization kit with the low-profile rail element by bringing in contact the planar opposite surface of mobile patella trial element with the second surface of the low-profile rail element. In some embodiments, in order to allow the two elements to stay in close contact, while still allowing the sliding of the mobile patella trial element relative to the low-profile rail element, the connection between the two mentioned elements features some type of mechanical restraint connection (e.g., a T-rail, a dovetail, a detent, etc.). In some embodiments, the surgeon then attaches aforementioned assembled instrumented patella trial component to the resected native patella by placing the first surface of the low-profile rail element in contact with the resected surface of the native patella. In some embodiments, in order to maintain the instrumented patella trial component attached with the resected native patella during the following steps, the first surface of the low-profile rail element features a plurality of spikes intended to securely connect with the resected surface of the native patella, as described above with reference to Figure 2B. In some embodiments, the low-profile rail element has an elongated aspect. In some embodiments, the long axis of the low-profile rail element is positioned along the long axis of the resected patella. Figure 6 shows the second characterization kit as positioned in this manner. Generally, the long axis of the patella is aligned along the mediolateral axis. Anthropometric studies have established that the mediolateral width of a patella is generally larger than its height, with the average width-to-height ratio around 1.25 regardless of gender or race. Figure 6 also schematically illustrates the wireless communication between the second characterization kit and the exemplary inventive computing device, by which characterization data may be transmitted to the exemplary inventive computing device.

In some embodiments, next, the surgeon performs a trial reduction by positioning the resurfaced resected native patella (i.e., instrumented as described above) in its physiological position. In some embodiments, the trial reduction is performed before the preparation of the distal femur. In some embodiments, the trial reduction is performed after the preparation of the distal femur. In some embodiment, the patella is positioned by aligning it with the trochlea groove of the native femur (if before the preparation of the distal femur) or the femoral trial component (if after the preparation of the distal femur). In some embodiments, after the patella is positioned, the surgeon simulates flexion(s) of the knee joint. In some embodiments, as a direct consequence of the compressive contact between the articular surface of the mobile patella trial element and the concave trochlea of the native femur or the femoral trial component, the sensor array characterizes at least one of (a) the force occurring between the surface of the mobile patella trial element and the second surface of the low-profile rail element, (b) the location of the center of the said force, (c) the magnitude of the moment resisted by the mobile patella trial element (both along the mediolateral axis and the internal-external axis), and (d) the mediolateral position of the mobile patella trial element relative to the low-profile rail element. In some embodiments, the sensor array comprises at least two rows of force sensitive aspects. In some embodiments, force is recorded at each position within the array by interpreting a change in impedance (e.g., resistive, capacitive, or inductive). In some embodiments, the sensor output is interpreted by the processing module. In some embodiments, the processing module calculates a data array including forces, moment, and spatial data. In some embodiments, the processing module analyzes the data in order to determine the aforementioned desired values (e.g., force, center of force, magnitude of moment resisted by the patella). In some embodiments, the processing module performs such computations using known techniques.

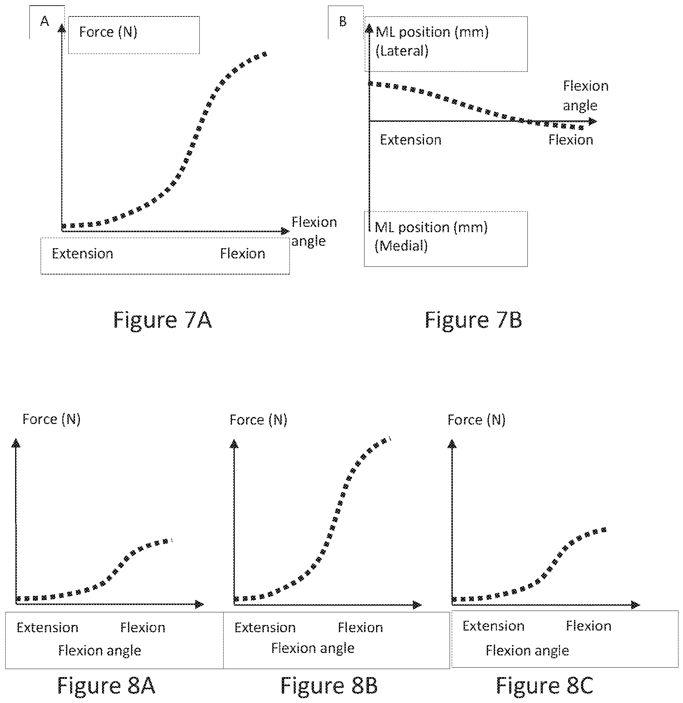

As noted, in some embodiments, the characterization of the patellofemoral joint with the second characterization kit can be obtained either (1) after preparation of the patella but before the preparation of the femur, (2) after preparation of the patella and after the preparation of the femur, or (3) after preparation of the patella but before the preparation of the femur as well as after preparation of the patella and after the preparation of the femur (i.e., both (1) and (2)), in order to monitor the impact of the femoral preparation on the patellofemoral joint. In some embodiments, the surgeon initiates the characterization with the leg in extension and stops it with the leg in full flexion (or vice versa). In some embodiments, data associated with said characterization is wirelessly transmitted to the computing device. In some embodiments, the computing device features a display system in order to display customized graphical user interfaces for the real-time visualization of the characterization. As a result, the surgeon obtains an accurate recording of the patellofemoral joint in terms of load intensity, load pattern and location over the range of motion from full extension to full flexion (assessed as a function of acquisition time). Figures 7A and 7B show exemplary displays that may result from a characterization as described above. Figure 7A shows a display of compressive force as a function of flexion angle, while Figure 7B shows a display of mediolateral position as a function of flexion angle.

In some embodiments, by comparing two characterization data sets, the surgeon can elect to follow the guidance from the computing device and, if needed, perform change(s) to the patellofemoral joint by changing the geometry of the mobile patella trial element or modifying its position. Some examples of changes intended to illustrate the advantages of the characterization process of the patellofemoral joint per the proposed embodiment are described hereinafter. These examples shall not be considered as limiting the breadth, scope, or applicability of the proposed embodiment.

### Example #1

In a first example, the surgeon performs a hybrid characterization of the patellofemoral joint with the second characterization kit (i.e., after the preparation of the patella but before the preparation of the femur). Next, subsequent to the preparation of the femur, the surgeon places the trial femoral component on the prepared distal femur and then performs a prosthetic characterization of the patellofemoral joint with the second characterization kit (i.e., after preparation of both the patella and the femur). Figure 8A shows a graph of compressive force as a function of flexion angle resulting from the first characterization (Input), while Figure 8B shows a graph of compressive force as a function of flexion angle resulting from the second characterization (Output₁). In this example, a review of the two sets of characterization data reveals a sharp increase in intensity of the force from the hybrid characterization (Input) to the second characterization (Output₁). Consequently, in this example, the computing device displays a quantitative assessment and suggests to reduce the thickness of the mobile patella trial element in order to decrease the force. Based on this suggestion the surgeon may elect to use a mobile patella trial element associated with the same outer diameter as previously selected, but with a thinner thickness. Following such a change, the surgeon performs a subsequent prosthetic characterization of the patellofemoral joint in order to evaluate the impact of the change. Figure 8C shows a graph of compressive force as a function of flexion angle resulting from this third characterization (Output₂). It may be seen that the Output₂ graph more closely resembles the Input graph than does the Output₁ graph.

### Example #2

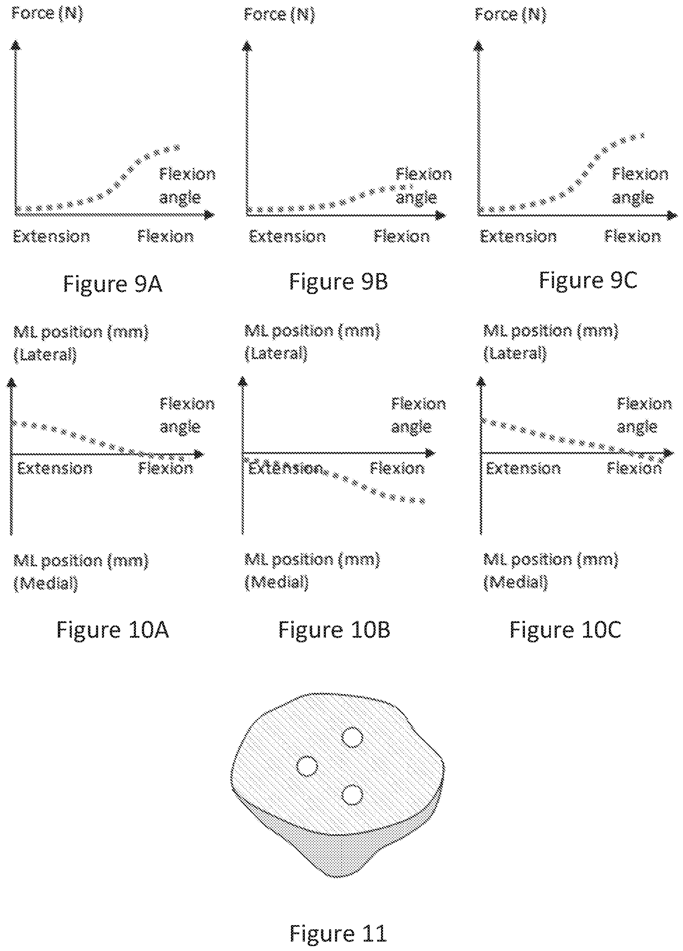

In a second example, the surgeon performs a hybrid characterization of the patellofemoral joint with the second characterization kit (i.e., after the preparation of the patella but before the preparation of the femur). Subsequent to the preparation of the femur, the surgeon places the trial femoral component on the prepared distal femur and then performs a prosthetic characterization of the patellofemoral joint with the second characterization kit (i.e., after preparation of both the patella and the femur). Figure 9A shows a graph of compressive force as a function of flexion angle resulting from the first characterization (Input), while Figure 9B shows a graph of compressive force as a function of flexion angle resulting from the second characterization (Output₁). In this example, a review of the two sets of characterization data reveals a sharp decrease in intensity of the force the hybrid characterization (Input) to the second characterization (Output₁). Consequently, in this example, the computing device displays a quantitative assessment and suggests to increase the thickness of the mobile patella trial element in order to increase the force. Based on this suggestion the surgeon may elect to use a mobile patella trial element associated with the same outer diameter as previously selected, but with a thicker thickness. Following such a change, the surgeon performs a subsequent prosthetic characterization of the patellofemoral joint in order to evaluate the impact of the change. Figure 9C shows a graph of compressive force as a function of flexion angle resulting from this third characterization (Output₂). It may be seen that the Output₂ graph more closely resembles the Input graph than does the Output₁ graph.

### Example #3

In this example, the surgeon performs a hybrid characterization of the patellofemoral joint with the second characterization kit (i.e., after the preparation of the patella but before the preparation of the femur). Subsequent to the preparation of the femur, the surgeon places the trial femoral component on the prepared distal femur and then a prosthetic characterization of the patellofemoral joint with the second characterization kit (i.e., after preparation of both the patella and the femur). Figure 10A shows a graph of mediolateral position of the mobile patella trial element as a function of flexion angle resulting from a first characterization performed with the second characterization kit (Input), while Figure 10B shows a graph of the mediolateral position of the mobile patella trial element as a function of flexion angle resulting from a second characterization performed with the second characterization kit (Output₁). In this example, a review of the two sets of characterization data reveals a sharp medial shift of the mediolateral position of the patella from the first characterization to the second characterization. Consequently, in this example, the computing device displays a quantitative assessment and suggests shifting the femoral component towards the lateral aspect or to select a more medial position of the patella implant relative to the resected native patella. Following such a change, the surgeon performs a subsequent prosthetic characterization of the patellofemoral joint in order to evaluate the impact of the change. Figure 10C shows a graph of mediolateral position of the mobile patella trial element as a function of flexion angle resulting from a third characterization performed with the second characterization kit (Output₂). It may be seen that the Output₂ graph more closely resembles the Input graph than does the Output₁ graph.

In some embodiments, once a satisfactory prosthetic characterization of the patellofemoral joint has been achieved, the surgeon uses the patella implant matching the configuration of the instrumented trial component in term of diameter, thickness and angulation, and then completes the preparation of the resected native patella by drilling at least one hole intended to receive the pegs of the selected patella implant. In some embodiments, one hole is drilled. In some embodiments, more than one hole is drilled. Figure 11 shows a resected native patella after having holes drilled therein in this manner. In some embodiments, the surgeon implants the selected patella implant on the prepared resected native patella by fitting the at least one peg of the implant with the corresponding at least one hole of the prepared patella. In some embodiments, surgical PMMA cement can be used to improve the bonding between the selected patella implant and the prepared resected native patella.

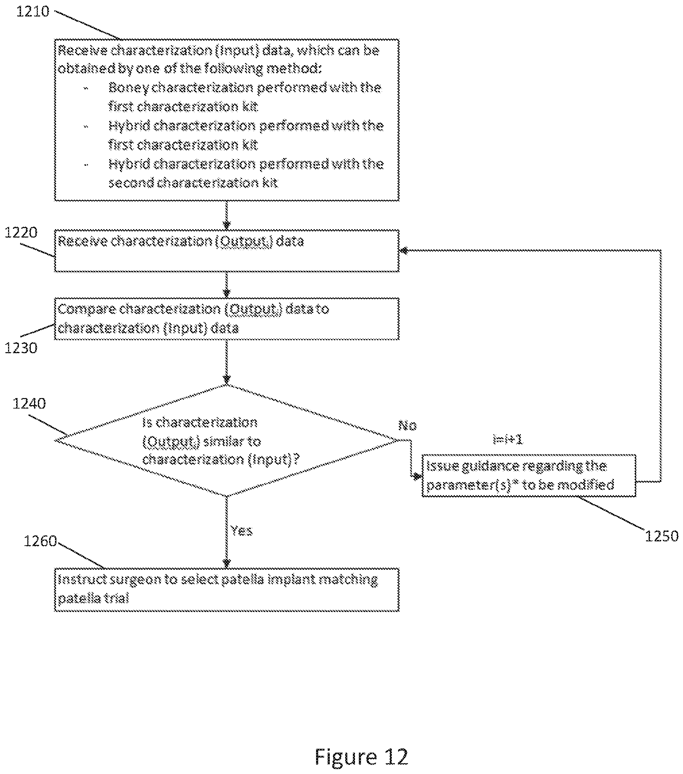

Figure 12 illustrates a flowchart of an exemplary method that may be performed by an exemplary inventive computing device. In step 1210, the exemplary inventive computing device (i.e., as described above with reference to the elements of the exemplary patella kit) receives an Input characterization data set. In some embodiments, the Input characterization data set relates to a boney characterization obtained using the first characterization kit (i.e., as described above with reference to Figures 3A and 3B). In some embodiments, the Input characterization data set relates to a hybrid characterization obtained using the first characterization kit described above. In some embodiments, the Input characterization data set relates to a hybrid characterization obtained using the second characterization kit as described above. After step 1210, a practitioner (e.g., a surgeon) may put the femoral trial, tibial trial, and instrumented patella trial in place. In some embodiments, the femoral trial and tibial trial are placed in accordance with known techniques. In some embodiments, the instrumented patella trial is placed as described above with reference to Figures 5 and 6. Following placement of the femoral trial, tibial trial and instrumented patella trial, the patellofemoral and femorotibial joints are reduced to their native positions.

In step 1220, the exemplary inventive computing device receives an Outputᵢ characterization data set. In some embodiments, the Outputᵢ characterization data set results from the performance a prosthetic characterization of the knee joint that has been prepared as described above (i.e., has had an instrumented patella trial placed therein). As used herein with reference to the data set Outputᵢ, i is a variable representing the quantity of iterations that have been made.

In step 1230, the exemplary inventive computing device compares the characterization data set Outputᵢ to the Input characterization data set to assess the similarity between these data sets. In some embodiments, the similarity between the two data sets is evaluated using known statistical method (e.g., a Kolmogorov-Smirnov test, a Ramer-Douglas-Peucker algorithm, a Procrustes analysis, a Frechet distance, etc.). In some embodiments, the similarity between the two curves is assessed by evaluating the distance between the two curves. In some embodiments, the similarity between the two curves is assessed by evaluating the surface area between the two curves. In some embodiments, for a given value along the X-axis (i.e., flexion angle), the analysis compares the corresponding values along the Y-axis (e.g., the load) between the Input and Outputᵢ data sets. In some embodiments, a metric of similarity will be lower when two curves are more similar, and will be higher when two curves are more different.

Generally speaking, the patellofemoral joint tends to be lax in extension and under compressive load as the knee flexes above 60 degrees of flexion. Consequently, in some embodiments, the comparison described above focuses on the range of flexion angles ranging from 60 degrees of flexion to 150 degrees of flexion. In some embodiments, the comparison includes only the range of flexion angles ranging from 60 degrees of flexion to 150 degrees of flexion. In some embodiments, the range of flexion angles ranging from 60 degrees of flexion to 150 degrees of flexion is weighted more heavily than flexion angles outside this range. In some embodiments, the surgeon can modify this range.

Continuing to refer to Figure 12, in step 1240, the exemplary inventive computing device assesses the similarity between the Input and Outputᵢ data sets to determine whether they are sufficiently similar. In some embodiments, this assessment is made by comparing a metric of similarity (e.g., one determined as described above with reference to step 1253) to a threshold value. In some embodiments, the threshold value is a relative value (e.g., 250 Newtons, 5 millimeters, etc.). In some embodiments, the threshold value is a percentage (e.g., 25%). In some embodiments, the threshold value is predetermined. In some embodiments, the threshold value is user-configurable. If the Input and Outputᵢ data sets are not sufficiently similar (i.e., a similarity metric describing the difference between the two exceeds a threshold value), the method 1200 proceeds to step 1250. If the Input and Outputᵢ data sets are sufficiently similar (i.e., a similarity metric describing the difference between the two is less than a threshold value), the method 1200 proceeds to step 1260.

In step 1250, the exemplary inventive computing device issues guidance regarding at least one parameter of the patella trial to be modified. In some embodiments, the at least one parameter includes a size of the patella trial. In some embodiments, the at least one parameter includes a thickness of the patella trial. In some embodiments, the guidance includes a suggestion to use a thinner implant if an Outputᵢ data set representing compressive force is greater than an Input data set representing compressive force. In some embodiments, the guidance includes a suggestion to use a thicker implant if an Outputᵢ data set representing compressive force is less than an Input data set representing compressive force. In some embodiments, the at least one parameter includes guidance regarding an angulation of the patella trial. In some embodiments, the guidance includes a suggestion to modify an angulation of the mobile patella trial element if an Outputᵢ data set representing interior-exterior rotation of the mobile patella trial varies from an Input data set representing interior-exterior rotation of the native patella. In some embodiments, the at least one parameter includes guidance regarding the positioning of the patella implant. In some embodiments, guidance includes a suggestion to select a more medial position of the patella implant relative to the resected native patella if an Outputᵢ data set representing the mediolateral position of the mobile patella trial element indicates a more lateral position than an Input data set representing the position of the native patella. In some embodiments, guidance includes a suggestion to select a more lateral position of the patella implant relative to the resected native patella if an Outputᵢ data set representing the mediolateral position of the mobile patella trial element indicates a more medial position than an Input data set representing the position of the native patella. In some embodiments, the at least one parameter includes guidance regarding the position of the femoral component along the mediolateral axis. In some embodiments, the at least one parameter includes guidance regarding a combination of more than one of the thickness, angulation, or position of the patella implant. Following step 1250, the surgeon implements the guidance provided by the exemplary inventive computing device (e.g., replaces the existing trial with one of a different size). Subsequently, the variable i is incremented and the method returns to step 1220, discussed above.

If the exemplary inventive computing device has determined, in step 1240, that the Input and Outputᵢ data sets are sufficiently similar, then in step 1260, the exemplary inventive computing device instructs the surgeon to select a patella implant matching the patella trial that was in place for the characterization that resulted in the data set Outputᵢ (i.e., a patella implant having the same size and/or thickness and/or angulation). Following step 1260, the method 1200 is complete. The surgeon may then implement the recommendation resulting from the performance of the method 1200. In some embodiments, this includes completing the preparation of the patient's resected patella (e.g., by drilling at least one hole as described above with reference to Figure 11, applying an adhesive, and/or any other desired preparations) and placing the selected implant using known techniques.

In some embodiments, rather than obtaining a patient-specific Input data set, the algorithm described herein may use historical characterization data as an Input data set. In some embodiments, historical characterization data may be based on a data set generally applicable to all patients. In some embodiments, historical characterization data may be based on a data set that is customized to the patient's demographic data (e.g., at least one factor such as gender, age, ethnicity, etc.).

In some embodiments, the low-profile rail element described above includes an inertial sensor (e.g., inertial gyroscope) in addition to the sensor array described above. In some embodiments, the inertial sensor is configured to track the angular tilt of the instrumented patella trial component during the trial reduction intended to provide the characterization of the patellofemoral joint. As previously mentioned, the resection of the native patella is subjective in nature and the orientation of the resection plane of the native patella may not always be optimal. In cases where there is an angular discrepancy between the actual resection plane and what would have been a preferred resection plane, it may be beneficial to use a patella implant with some angular compensation between the perimeter of the articular surface and the opposite planar surface (i.e., an angulation parameter). In embodiments in which the low-profile rail element includes an inertial sensor, in addition to the parameters described above, a characterization provides information regarding the angular tilt of the instrumented patella trial component during the trial reduction. In such embodiments, when there is a substantial tilt, the algorithm described above may recommend changing the angulation of the mobile patella trial element. An example of changes intended to illustrate the advantages of the characterization process of the patellofemoral joint per such an embodiment are described hereinafter. This example shall not be considered as limiting the breadth, scope, or applicability of the proposed embodiment.

### Example #4

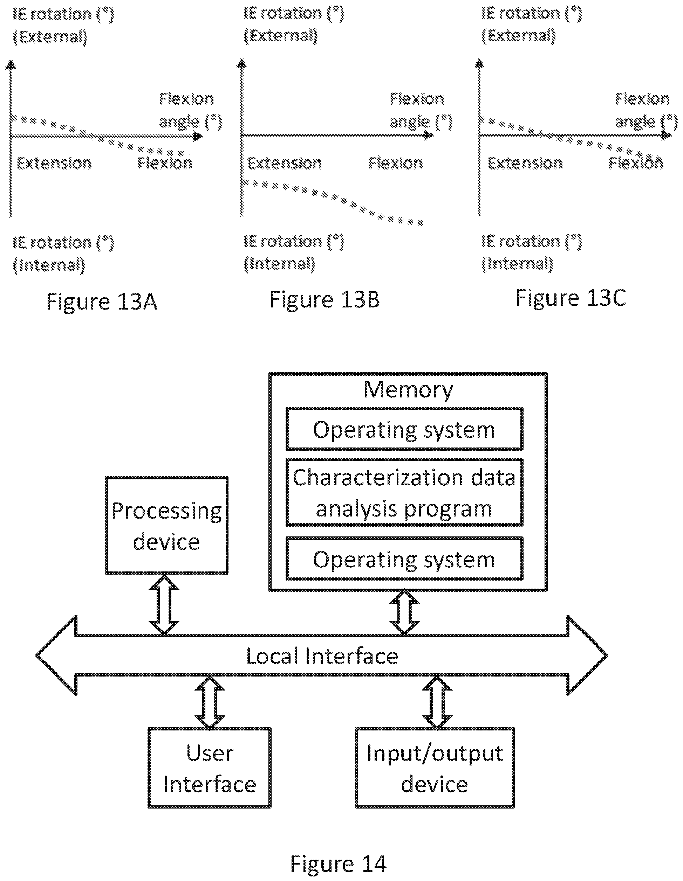

In a fourth example, the surgeon performs a hybrid characterization of the patellofemoral joint with the second characterization kit (i.e., after the preparation of the patella but before the preparation of the femur). Next, subsequent to the preparation of the femur, the surgeon places the trial femoral component on the prepared distal femur and then performs a prosthetic characterization of the patellofemoral joint with the second characterization kit (i.e., after preparation of both the patella and the femur). Figure 13A shows a graph of internal-external rotation as a function of flexion angle resulting from the first characterization performed with the second characterization kit (Input), while Figure 13B shows a graph of internal-external rotation of the mobile patella trial of the mobile patella trial as a function of flexion angle resulting from the second characterization performed with the second characterization kit (Outputi). In this example, a review of the two sets of characterization reveals a sharp tilt of the orientation of the instrumented patella trial component from the hybrid characterization (Input) to the second characterization (Output₁). Consequently, in this example, the computing device displays a quantitative assessment and suggests to modify the angulation of the mobile patella trial element in order to decrease the internal rotation. Based on this suggestion the surgeon may elect to use a mobile patella trial element associated with a wedged geometry (angulation sub-option) in order to compensate for the tilt. Following such a change, the surgeon can perform a subsequent prosthetic characterization of the patellofemoral joint in order to evaluate the impact of the change. Figure 13C shows a graph of internal-external rotation as a function of flexion angle resulting from this third characterization (Output₂). It may be seen that the Output₂ graph more closely resembles the Input graph than does the Output₁ graph.

Figure 14 schematically illustrates the elements of the exemplary inventive computing device. As shown in this figure, the exemplary inventive computing device includes a processing device, a memory, a user interface, and at least one input/output device, each of which is connected to a local interface (e.g., a bus). In some embodiments, the processing device includes a central processing unit ("CPU") or a semiconductor-based microprocessor (e.g., in the form of a microchip). In some embodiments, the memory includes any one of or a combination of volatile memory elements (e.g., RAM) and nonvolatile memory elements (e.g., hard disk, ROM, Flash, etc.). In some embodiments, the user interface includes the components with which a user interacts with the computing device, such as a keyboard, keypad, and/or display screen. In some embodiments, the user interface includes a display screen configured to provide guidance to a surgeon as described above. In some embodiments, the at least one input/output device is configured to facilitate communications with other devices. In some embodiments, the at least one input/output device includes a wireless data link configured to receive characterization data from the first and second characterization kits. In some embodiments, the memory includes programs (logic) including an operating system and a characterization data analysis program configured to analyze characterization data and provide recommendations as, for example, described above with reference to the exemplary method 1200. In some embodiments, the memory includes a database storing historical characterization data for use as Input characterization data.

In some embodiments, a sensing element including an inertial sensor can be leveraged to provide information regarding the flexion angles of the knee. For example, such a sensing element can accurately detect the flexion angles through the range of motion from full extension to full flexion positions and therefore limit the acquired data to the flexion angles of interest (e.g., from 60 degrees to 120 degrees of flexion).

In some embodiments, an instrumented patella trial component as described above is used in combination with a knee navigation system. Knee navigation is known to provide accurate information in terms of frontal and sagittal mechanical alignments. Therefore, in such embodiments, the information provided by a knee navigation system can be cross-referenced with the data acquired by the sensor array, so the characterization of the patellofemoral joint can be expressed in terms of flexion angle of the knee.

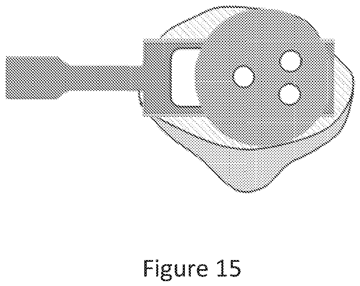

In some embodiments, an instrumented patella trial component as described above is arranged such that the positioning hole(s) for the pegs(s) of the patella implant can be drilled directly through the instrumented patella trial component. In some embodiments, such arrangements include at least one hole on the mobile patella trial component and at least one open window on the low-profile rail element. Figure 15 shows a patella trial component that is arranged in this manner.

In some embodiments, an instrumented patella trial component as described above includes a mechanism configured to selectively lock and unlock the translation of the mobile patella trial element relative to the low-profile rail element. In some embodiments, by locking this degree of freedom, the surgeon can better assess the behavior of the patella implant by simulating a given mediolateral position during the characterization of the patellofemoral joint.

In some embodiments, the contour (i.e., profile) of a mobile patella trial component is non-circular rather than circular. In some embodiments, a non-circular contour is either oval, oblong, or piriform (i.e., pear-shaped). In some embodiments, the number of degrees of freedom of the mobile patella trial element relative to the low-profile rail element may vary. In some embodiments, the number of degrees of freedom is zero. In some embodiments, the number of degrees of freedom is one. In some embodiments, the number of degrees of freedom is two. In some embodiments, the number of degrees of freedom is three. In some embodiments, a degree of freedom is rotational. In some embodiments, a degree of freedom is translational. In some embodiments, a degree of freedom is both rotational and translational.

Exemplary embodiments have been described above with specific reference to TKA with or without resurfacing of the patella. However, it will be understood by those of skill in the art that the concepts embodied by the exemplary embodiments are also applicable to other joints where a first characterization of the joint is performed, a second characterization is performed after at least one bone cut from the joint has been performed, and the comparison between the first and second characterization is leveraged to guide the selection of an implant from an implant kit.

In some embodiments, an exemplary patella kit system includes an optional first characterization kit, a second characterization kit, a computing device and an implant kit. In some embodiments, the first characterization kit is configured to perform characterization of the patellofemoral joint before resurfacing of the patella. In some embodiments, the second characterization kit is configured to perform characterization of the patellofemoral joint after resurfacing of the patella. In some embodiments, the computing device is configured to receive data from the first characterization kit and the second characterization kit. In some embodiments, based on analysis of the data, the computing device provides a surgeon with instructions and guidance in order to select an optimal patella implant from the patella kit and/or to optimally position it. In some embodiments, the instructions and guidance are based on the comparison of a characterization performed at an early stage during the surgical procedure (referred to herein as Input) with a characterization performed at a later stage with the trial components in place (referred to herein as Output). In some embodiments, in case of discrepancy between the two sets of characterization data, the computing device issues instructions in order to decrease the level of discrepancy. In some embodiments, assuming the surgeon elects to implement the change, he may then perform a subsequent characterization with the modified parameters in order to confirm the improvement.

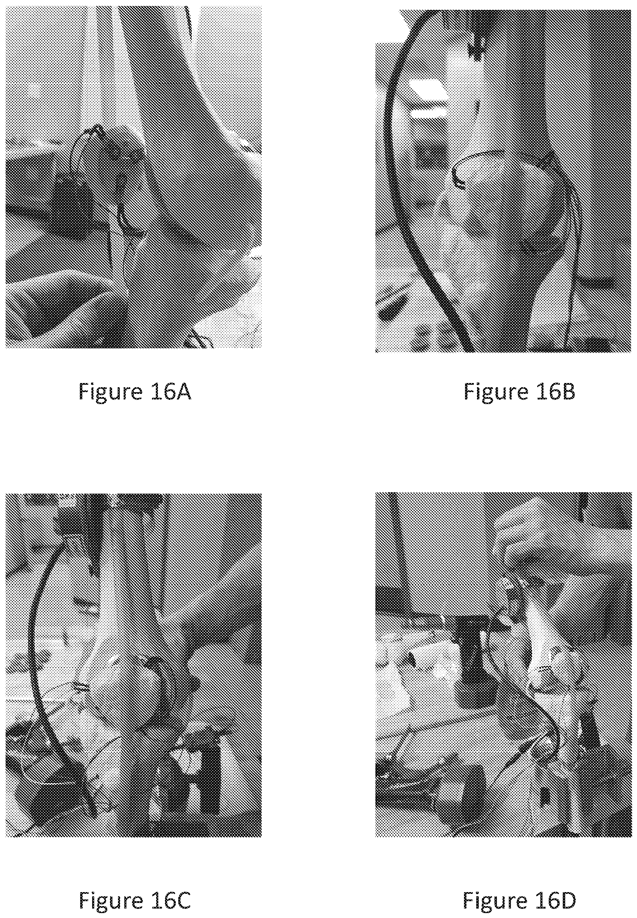

Referring now to Figures 16A-16D, the evaluation of a prototype of the second characterization kit described above with an anatomical model is shown in a configuration in which the patella trial does not translate with respect to the underlying rail. In some embodiments, as shown in Figure 16A, the second characterization kit includes three force sensors located between an instrumented patella baseplate and a patella trial (not shown in Figure 16A). In some embodiments, data recorded by the three force sensors is analyzed to compute the principal direction and magnitude of loading. Figure 16B shows an exemplary secondary characterization kit as used to perform an initial characterization of the patellofemoral joint after preparation of the patella and before preparation of the distal femur. Figure 16C shows the exemplary secondary characterization kit as prepared for use to perform an output characterization after preparation of the distal femur. Figure 16D shows the use of the exemplary secondary characterization kit to perform an output characterization, with the anatomical model shown being moved through a simulated range of motion.

In some embodiments, an initial characterization of the patellofemoral joint (Input) is performed based on the native joint using the first characterization kit described above. In some embodiments, an initial characterization of the patellofemoral joint (Input) is performed before the preparation of the patella and after the preparation of the distal femur using the first characterization kit described above. In some embodiments, an initial characterization of the patellofemoral joint (Input) is performed after the preparation of the patella and before the preparation of the distal femur using the second characterization kit described above. In some embodiments, an initial characterization of the patellofemoral joint (Input) is determined based on a data set of historical characterizations. In some embodiments, subsequent characterizations (Outputᵢ) are performed using the second characterization kit as described above.

In some embodiments, regardless of the characterization kit (i.e., first characterization kit or second characterization kit) and the characterization sequence (i.e., input or output), the characterization of the patellofemoral joint relates to the joint reaction force through a simulated range of motion (e.g., from extension to flexion) between the patella and the distal femur. In some embodiments, using a dedicated profiler, the user surgeon can define the sensitivity of the comparison between the two characterizations. In some embodiments, the surgeon can pre-define a range of flexion of particular interest for the comparison (e.g., from 30° to 100° of flexion). In some embodiments, the surgeon can pre-define a desired similarity between the two characterizations according to a threshold tolerance T. In some embodiments, the threshold is expressed as a value. In some embodiments, the threshold is expressed as a percentage. In some embodiments, the surgeon can pre-define other information regarding the scope of implants available at the time of surgery (e.g., thickness options, angulation options, or thickness and angulation options).

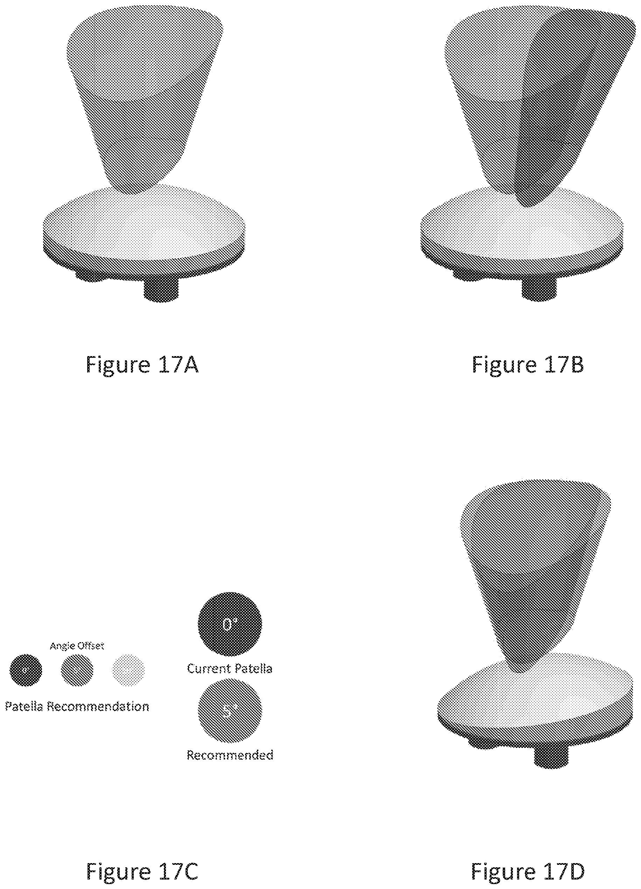

In some embodiments, as the patellofemoral joint is moved through the simulated range of motion, the instantaneous joint reaction forces are recorded. In some embodiments, the joint reaction forces are analyzed to produce an overall 3D envelope based on the sum of the collected instantaneous joint reaction forces. It will be apparent to those of skill in the art that the 3D envelopes shown in some of the Figures to be described hereinafter represent only one mode of representation of the recorded force information, and that other types of two-dimensional or three-dimensional data visualization (e.g., histograms, maps, etc.) are also suitable. In some embodiments, the 3D envelope represents the characterization of the patellofemoral joint at a given stage along the procedure. According to one example of a graphical user interface, the 3D envelope is represented with reference to the instrumented patella trial (e.g., as shown in Figure 17A). In some embodiments, the 3D envelope represents both magnitude of force (e.g., by the size of the 3D envelope) and the orientation of force (e.g., by the position and orientation of the 3D envelope). In some embodiments, guidance provided to the surgeon by a system operating in accordance with the method shown in Figure 12 is provided based on the comparison between two characterizations (i.e., Outputᵢ vs. input) in terms of orientation and amplitude.

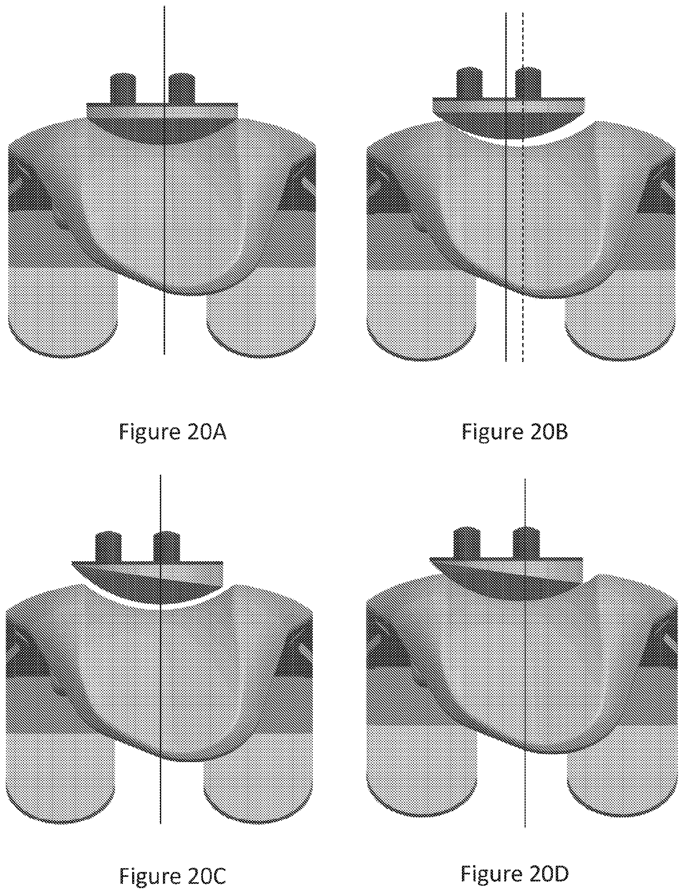

In some embodiments, each 3D envelope is defined by a singular resultant force representing an "averaging" of the instantaneous joint reaction forces, where the resultant force is defined by its orientation as well as its amplitude. In some embodiments, the first singular resultant force from the first envelope associated with the first characterization of the patellofemoral joint and the subsequent singular resultant force from the subsequent envelope associated with the subsequent characterization of the patellofemoral joint are compared to one another based on their respective orientation and amplitude. In some embodiments, a numerical analysis technique is applied to determine which component(s) (e.g., orientation, amplitude, or both orientation and amplitude) shows a statistically significant difference with respect to flexion angle. In some embodiments, the numerical analysis technique is a regression analysis. In some embodiments, the regression analysis includes a least squares analysis. In some embodiments, the computed differences between the first and subsequent characterizations of the patellofemoral joint (if statistically significant) are used to provide guidance to the surgeon in determining an appropriate patellar geometry. In some embodiments, the thickness of the patella trial used in a given characterization will typically impact mainly the amplitude of the joint force reaction, whereas the angulation aspect and the associated clocking of the patella (i.e., axial rotation of the patella trial about an axis perpendicular to the resected patella) will typically mainly impact the orientation of the joint force reaction. As shown in Figures 20A-20D, angulation of the patellar implant effectively creates a transverse alignment offset between the patellar and femoral components. Figure 20A shows well-aligned patellofemoral contact. As shown in Figure 20B, a lateral misalignment of the patella with respect to the condylar groove of the femoral component will produce edge loading onto the patella, thereby altering the orientation of the resultant force measured during second characterization compared to the first characterization data set. As shown in Figure 20C, an angulated patella can compensate for such misalignment conditions to restore proper patellofemoral contact. In particular, an angulated patella can be clocked (i.e., rotated) to compensate for a range of misalignment conditions. Figure 20D shows the reassembled joint demonstrating proper patellofemoral contact. In some embodiments, based on the above considerations, guidance is provided to the surgeon as described above with reference to Figure 12 and/or as described hereinafter.

In some embodiments, each instantaneous measured joint reaction force is associated with the degree of flexion of the knee joint at the time of the measurement. In some embodiments, for each angle of flexion of interest, the first joint reaction force associated with the first characterization of the patellofemoral joint and the subsequent joint reaction force associated with the subsequent characterization of the patellofemoral joint are compared to one another based on their respective orientation and amplitude. In some embodiments, a numerical analysis technique is applied to determine which component(s) (e.g., orientation, amplitude, or both orientation and amplitude) shows a statistically significant difference with respect to flexion angle. In some embodiments, the numerical analysis technique is a regression analysis. In some embodiments, the regression analysis includes a least squares analysis. In some embodiments, the computed differences between the first and subsequent characterizations of the patellofemoral joint (if statistically significant) are used to provide guidance to the surgeon in determining an appropriate patellar geometry. In some embodiments, these differences are averaged in order to provide guidance to the surgeon. In some embodiments, the thickness of the patella trial used in a given characterization will typically impact mainly the amplitude of the joint force reaction, whereas the angulation aspect and the associated clocking of the patella (i.e., axial rotation of the patella trial according to an axis perpendicular to the resected patella) will typically impact mainly the orientation of the joint force reaction. Based on this consideration, basic simulation is used to provide the guidance to the user surgeon (e.g., as described above).

In some embodiments, if it is determined that there is a statistically significant difference between the first characterization and the subsequent characterization, guidance is based on the following principles. In some embodiments, if the amplitude of the joint force associated with the subsequent characterization (Outputᵢ) is greater than the amplitude of the joint force associated with the initial characterization (Input), then the guidance is issued to decrease the thickness of the patella trial. In some embodiments, if the amplitude of the joint force associated with the subsequent characterization (Outputᵢ) is less than the amplitude of the joint force associated with the initial characterization (Input), then the guidance is issued to increase the thickness of the patella trial. In some embodiments, if the orientation of the joint force associated with the subsequent characterization (Outputᵢ) is different than the orientation of the joint force associated with the initial characterization (Input), then the guidance is issued to compensate for this offset discrepancy by changing the angulation and the associated clocking of the patella trial (i.e., axial rotation of the patella trial according to an axis perpendicular to the resected patella). In some embodiments, a different orientation is deemed to occur if the two orientations differ by more than a threshold amount. In some embodiments, the threshold amount is 5 degrees. In some embodiments, guidance is determined by analyzing the angular offset between the joint force associated with the subsequent characterization (Outputᵢ) and the joint force associated with the initial characterization (Input) to provide guidance about the modified angulation of the patella trial. In some embodiments, both the Output and Input data sets would consist of an array of three-dimensional force vectors (e.g., in a cartesian or polar coordinate system) with respect to flexion angle of the joint during the characterization. In some embodiments, a linear regression is performed on the computed difference between the Output and Input data sets, and the impact of orientation and force magnitude (calculated by deconvoluting the resultant force vectors) are tested for statistical significance. In some embodiments, a statistically significant difference in orientation is understood to imply edge loading of the patella, and a translational adjustment of the patella with respect to the femoral condylar groove is instructed. In some embodiments, an instruction is provided to use an angulated patellar trial that is clocked (i.e., oriented) in a direction opposite to the loaded edge. This would be achieved by providing guidance to the surgeon that suggests the use of an angulated patella, oriented (i.e. clocked) in the direction opposite to the loaded edge. In some embodiments, a statistically significant difference in the force magnitude is understood to imply that an adjustment in patella thickness is necessary. In some embodiments, the surgeon is provided with guidance to use a patellar trial with a reduced thickness (if Output is greater than Input) or increased thickness (if Output is less than Input). In some embodiments, the above principles are considered individually (i.e., only considering thickness adjustment or only considering angulation adjustment) or are considered in combination (i.e., considering both thickness adjustment and angulation adjustment) depending on the comparison between the characterizations and depending on the available scope of different patella trials.

Referring now to Figures 17A-17D, an exemplary analysis is shown. In some exemplary kits, the patellar implants are available under different diameters and under different angulations (e.g., 0°, +5°, and +10°) for any given diameter. In some embodiments, after the incision, the surgeon resurfaces the patella and places a first patella trial (e.g., 0° angulation) of the second characterization kit against the resected patella. Next, in some embodiments, the surgeon reduces the joint and performs a first characterization of the patellofemoral joint (Input) in order to collect the joint reaction forces between the attached patella trial (e.g., 0° angulation) and the native distal femur. Figure 17A shows a 3D envelope as described above for such a first characterization. Next, in some embodiments, the surgeon performs the preparation of the distal femur and places the corresponding femoral component in place. Next, in some embodiments, the surgeon (re-)reduces the joint and performs a second characterization of the patellofemoral joint (Outputi) in order to collect the joint reaction forces between the attached patella trial (e.g., 0° angulation) and the femoral component. Figure 17B shows a 3D envelope as described above for such a second characterization along with the 3D envelope for the first characterization as shown in Figure 17A for comparison. In some embodiments, the reaction forces (i.e., vectors) are deconvoluted into their constituent orthogonal planar or polar elements according to standard vector analysis. In some embodiments, a statistical analysis is performed to determine if the difference between the Input and Output₁ data for each constituent was statistically significant. In some embodiments, the statistical analysis is a regression analysis. In some embodiments, for the example shown in Figures 17A and 17B, the analysis would indicate that the difference in orientation between the first characterization of the patellofemoral joint and second characterization is statistically significant, and therefore would suggest a non-zero angular offset oriented to counter the calculated difference in orientation, as shown in Figure 17C. Next in some embodiments, the user surgeon follows the guidance, attaches the recommended patella trial (e.g., a trial with 5° angulation) to the resected patella and performs a subsequent characterization of the patellofemoral joint (Output₂) in order to collect the joint reaction forces between the newly attached patella trial (e.g., 5° angulation) and the femoral component. Figure 17D shows a 3D envelope as described above for such a subsequent characterization along with the 3D envelope for the first characterization as shown in Figure 17A for comparison. As may be seen, following the performance of the exemplary method, the two envelopes are substantially aligned with one another.

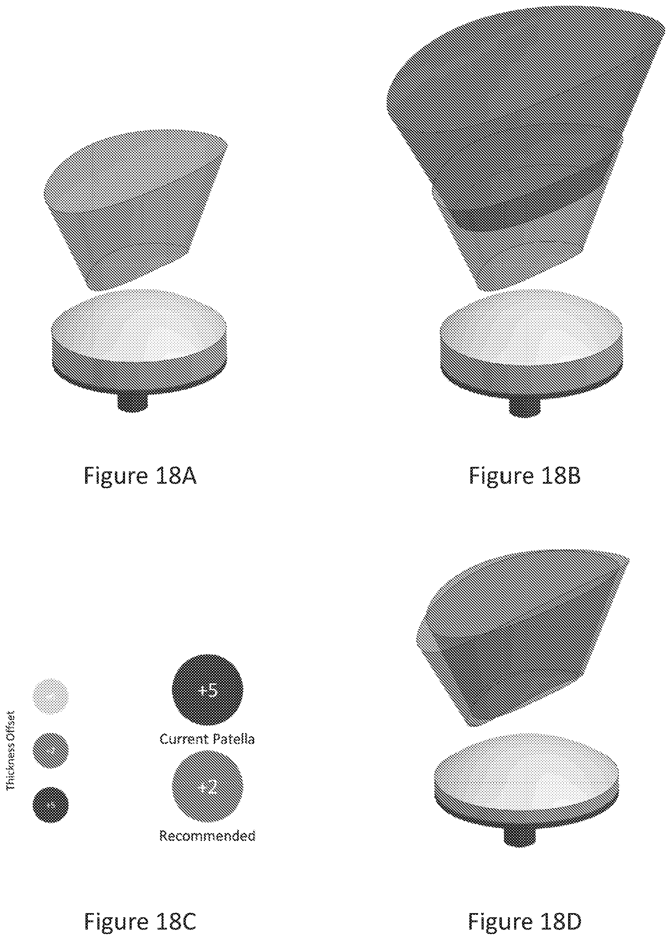

Referring now to Figures 18A-18D, an exemplary analysis is shown. In some exemplary kits, the patellar implants are available under different diameters as well as different thicknesses (e.g., 0 mm, +2mm, and +5mm) for any given diameter. In some embodiments, after the incision, the user surgeon resurfaces the patella and places a first patella trial (e.g., +5mm thickness) of the second characterization kit against the resected patella. Next, in some embodiments, the user surgeon reduces the joint and performs a first characterization of the patellofemoral joint (Input) in order to collect the joint reaction forces between the attached patella trial (e.g., +5 mm thickness) and the native distal femur. Figure 18A shows a 3D envelope as described above for such a first characterization. Next, in some embodiments, the user surgeon performs the preparation of the distal femur and places the corresponding femoral component in place. Next, in some embodiments, the user surgeon (re-)reduces the joint and performs a second characterization of the patellofemoral joint (Output₁) in order to collect the joint reaction forces between the attached patella trial (e.g., +5 mm thickness) and the femoral component. Figure 18B shows a 3D envelope as described above for such a second characterization along with the 3D envelope for the first characterization as shown in Figure 18A for comparison. In some embodiments, the reaction forces (i.e., vectors) are deconvoluted into their constituent orthogonal planar or polar elements according to standard vector analysis. In some embodiments, the constituent elements are analyzed to determine if the difference between the Input and Output₁ data for each constituent was statistically significant. In some embodiments, the analysis is a statistical analysis. In some embodiments, the analysis is a regression analysis. In some embodiments, for the example shown in Figure 18B, the analysis indicates that the difference in amplitude between the first characterization of the patellofemoral joint and second characterization is statistically significant, and therefore suggests a reduced patella thickness offset to counter the increased amplitude of the Output₁ characterization as compared to the Input characterization, as shown in Figure 18C. Next, in some embodiments, the user surgeon follows the guidance, attaches the recommended patella trial (e.g., +2 mm thickness) to the resected patella, and then performs a subsequent characterization of the patellofemoral joint (Output₂) in order to collect the joint reaction forces between the newly attached patella trial (e.g., +2 mm thickness) and the femoral component. Figure 18D shows a 3D envelope as described above for such a subsequent characterization along with the 3D envelope for the first characterization as shown in Figure 18A for comparison. As may be seen, following the performance of the exemplary method, the two envelopes are substantially aligned with one another.

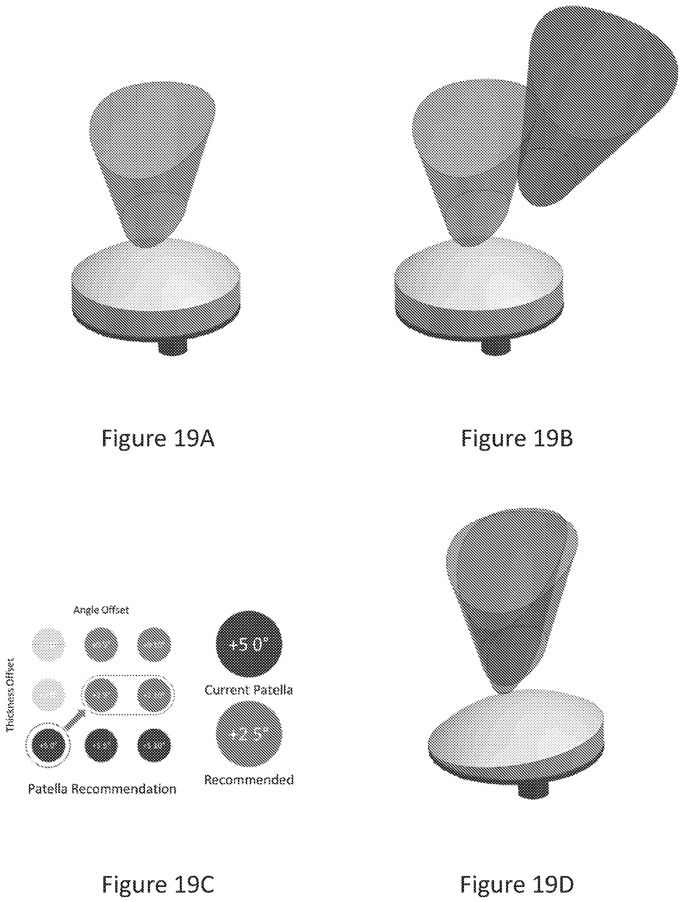

Referring now to Figures 19A-19D, an exemplary analysis is shown. In some exemplary kits, the patellar implants are available under different diameters as well as different angulations (e.g., 0°, +5°, and +10°) and thicknesses (e.g., 0 mm, +2mm, and +5mm) for any given diameter. In some embodiments, after the incision, the user surgeon resurfaces the patella and places the patella trial (e.g., +5mm thickness, 0° angulation) of the second characterization kit against the resected patella. Next, in some embodiments, the user surgeon reduces the joint and performs a first characterization of the patellofemoral joint (Input) in order to collect the joint reaction forces between the attached patella trial (e.g., +5mm thickness, 0° angulation) and the native distal femur. Figure 19A shows a 3D envelope as described above for such a first characterization. Next, in some embodiments, the user surgeon performs the preparation of the distal femur and places the corresponding femoral component in place. Next, in some embodiments, the user surgeon (re-)reduces the joint and performs a second characterization of the patellofemoral joint (Output₁) in order to collect the joint reaction forces between the attached patella trial (e.g., +5mm thickness, 0° angulation) and the femoral component. Figure 19B shows a 3D envelope as described above for such a second characterization along with the 3D envelope for the first characterization as shown in Figure 19A for comparison. In some embodiments, the reaction forces (i.e., vectors) are deconvoluted into their constituent orthogonal planar or polar elements according to standard vector analysis. In some embodiments, the constituent elements are analyzed to determine if the difference between the Input and Output₁ data for each constituent was statistically significant. In some embodiments, the analysis is a statistical analysis. In some embodiments, the analysis is a regression analysis. In some embodiments, for the Example shown in Figure 19B, the analysis indicates that the difference in orientation and amplitude between the first characterization of the patellofemoral joint and second characterization is statistically significant, and therefore suggests a non-zero angular offset a and reduced thickness offset to counter the calculated differences in orientation and amplitude, respectively, as shown in Figure 19C. Next, in some embodiments, the user surgeon follows the guidance, attaches the recommended patella trial (e.g., +2 mm thickness, 5° angulation) to the resected patella, and then performs a subsequent characterization of the patellofemoral joint (Output₂) in order to collect the joint reaction forces between the newly attached patella trial (e.g., +2 mm thickness. 5° angulation) and the femoral component. Figure 19D shows a 3D envelope as described above for such a subsequent characterization along with the 3D envelope for the first characterization as shown in Figure 19A for comparison. As may be seen, following the performance of the exemplary method, the two envelopes are substantially aligned with one another.

The exemplary embodiments described above make specific reference to the selection of implants suitable for implantation in the knee joint of a patient. However, it will be apparent to those of skill in the art that the systems and methods described herein may be adapted for use in other joints of humans and other mammals.

While a number of embodiments of the present invention have been described, it is understood that these embodiments are illustrative only, and not restrictive, and that many modifications may become apparent to those of ordinary skill in the art. For example, all dimensions discussed herein are provided as examples only, and are intended to be illustrative and not restrictive.

## Claims

1. A system for use in joint repair surgery, the system comprising:
- a sensing device configured for implantation within a joint of a patient, the sensing device including at least one sensor that is configured to record at least one reaction force within the joint while the joint is moved through a range of motion;
- a plurality of trial implants configured for trial implantation within the joint of the patient, each of the plurality of trial implants being configured to simulate one of a corresponding plurality of implants configured for permanent implantation within the joint; and
- a non-transient memory electronically storing computer executable program code, the computer executable program code configured such that a processor, when executing the program code, becomes a specifically programmed computing processor that is configured to perform at least the following operations:
- receiving, from the sensing device while the sensing device is positioned within a joint of a patient, a first data set representing a first series of reaction forces within the joint while the joint is moved through a range of motion during a first time period;
- receiving, from the sensing device while the sensing device is positioned within the joint of the patient, a second data set representing a second series of reaction forces within the joint while the joint is moved through the range of motion during a second time period, wherein a first one of the trial implants is attached within the joint during the first time period;
- calculating a difference between the second data set and the first data set; and
- providing an instruction based on the difference, wherein the instruction includes either (a) an instruction to select a second one of the trial implants if the difference exceeds a threshold, or (b) an instruction to proceed with an implant matching the first one of the trial implants if the difference does not exceed the threshold,
wherein the difference between the second data set and the first data set includes a different magnitude, and wherein the second one of the trial implants has a different thickness than the first one of the trial implants;
or
wherein the difference between the first data set and the second data set includes a positional offset, and wherein the second one of the trial implants has a different positional offset than the first one of the trial implants,
charactereized in that
the sensing device includes a rail, and wherein the at least one force sensor is movable linearly along the rail.

2. The system of claim 1,
further comprising a plurality of actual implants, each of the plurality of actual implants corresponding to one of the plurality of trial implants, wherein the plurality of trial implants are preferably patellar trials.

3. The system of claim 1 or 2,
wherein the threshold is either an absolute threshold or a relative threshold.

4. The system of one of claims 1 to 3,
wherein the sensing device comprises a plurality of force sensors, and wherein the difference between the second data set and the first data set includes a different orientation, and wherein the second one of the trial implants has a different angular offset than the first one of the trial implants.

5. The system of one of claims 1 to 4,
wherein the sensing device includes at least one of a resistance element, a capacitance element, a piezo-resistance element, a piezoelectric element, a hydraulic element, a pneumatic element, a magneto-elastic element, a vibration element, an optical element, or combinations thereof.

6. The system of one of claims 1 to 5,
wherein the operation of calculating the difference between the second data set and the first data set includes determining whether the difference between the second data set and the first data set is statistically significant.

7. The system of claim 6,
wherein a least squares method is used to determine whether the difference between the second data set and the first data set is statistically significant.

## Patentansprüche

1. System zur Verwendung in der Gelenkreparaturchirurgie,
wobei das System Folgendes aufweist:
- eine Erfassungsvorrichtung, die zur Implantation in ein Gelenk eines Patienten konfiguriert ist, wobei die Erfassungsvorrichtung mindestens einen Sensor aufweist, der so konfiguriert ist, dass er mindestens eine Reaktionskraft innerhalb des Gelenks aufzeichnet, während das Gelenk durch einen Bewegungsbereich bewegt wird;
- eine Vielzahl von Versuchsimplantaten, die für eine Versuchsimplantation in das Gelenk des Patienten konfiguriert sind, wobei jedes der Vielzahl von Versuchsimplantaten so konfiguriert ist, dass es eines aus einer entsprechenden Vielzahl von Implantaten simuliert, die für eine permanente Implantation in das Gelenk konfiguriert sind; und
- einen nichtflüchtigen Speicher, der elektronisch einen computerausführbaren Programmcode speichert, wobei der computerausführbare Programmcode so konfiguriert ist, dass ein Prozessor, wenn er den Programmcode ausführt, zu einem spezifisch programmierten Rechenprozessor wird, der so konfiguriert ist, dass er mindestens die folgenden Operationen durchführt:
- Empfangen eines ersten Datensatzes, von der Sensorvorrichtung, während die Sensorvorrichtung innerhalb eines Gelenks eines Patienten positioniert ist, der eine erste Reihe von Reaktionskräften innerhalb des Gelenks darstellt, während das Gelenk während einer ersten Zeitspanne durch einen Bewegungsbereich bewegt wird;
- Empfangen eines zweiten Datensatzes, von der Sensorvorrichtung, während die Sensorvorrichtung innerhalb des Gelenks des Patienten positioniert ist, der eine zweite Reihe von Reaktionskräften innerhalb des Gelenks darstellt, während das Gelenk während einer zweiten Zeitspanne durch den Bewegungsbereich bewegt wird, wobei ein erstes der Versuchsimplantate während der ersten Zeitspanne innerhalb des Gelenks angebracht ist;
- Berechnen einer Differenz zwischen dem zweiten Datensatz und dem ersten Datensatz; und
- Bereitstellen einer Anweisung auf der Grundlage der Differenz, wobei die Anweisung entweder (a) eine Anweisung zum Auswählen eines zweiten der Versuchsimplantate aufweist, wenn die Differenz einen Schwellenwert überschreitet, oder (b) eine Anweisung zum Fortfahren mit einem dem ersten der Versuchsimplantate entsprechenden Implantat aufweist, wenn die Differenz den Schwellenwert nicht überschreitet,
wobei der Unterschied zwischen dem zweiten Datensatz und dem ersten Datensatz eine unterschiedliche Größe aufweist, und wobei das zweite der Versuchsimplantate eine andere Dicke als das erste der Versuchsimplantate hat; oder
wobei der Unterschied zwischen dem ersten Datensatz und dem zweiten Datensatz einen Positionsversatz aufweist, und wobei das zweite der Versuchsimplantate einen anderen Positionsversatz als das erste der Versuchsimplantate aufweist,
**dadurch gekennzeichnet, dass**
- die Erfassungsvorrichtung eine Schiene aufweist und wobei der mindestens eine Kraftsensor linear entlang der Schiene beweglich ist.

2. System nach Anspruch 1, das ferner eine Vielzahl von tatsächlichen Implantaten aufweist,
wobei jedes der Vielzahl von tatsächlichen Implantaten einem der Vielzahl von Versuchsimplantaten entspricht, wobei die Vielzahl von Versuchsimplantaten vorzugsweise Patellaversuchsimplantate sind.

3. System nach Anspruch 1 oder 2,
wobei der Schwellenwert entweder ein absoluter Schwellenwert oder ein relativer Schwellenwert ist.

4. System nach einem der Ansprüche 1 bis 3,
wobei die Erfassungsvorrichtung eine Vielzahl von Kraftsensoren aufweist, und wobei der Unterschied zwischen dem zweiten Datensatz und dem ersten Datensatz eine unterschiedliche Ausrichtung aufweist, und wobei das zweite der Versuchsimplantate einen anderen Winkelversatz als das erste der Versuchsimplantate aufweist.

5. System nach einem der Ansprüche 1 bis 4,
wobei die Erfassungsvorrichtung mindestens eines Widerstandselements, eines Kapazitätselements, eines Piezo-Widerstandselements, eines piezoelektrischen Elements, eines hydraulischen Elements, eines pneumatischen Elements, eines magnetoelastischen Elements, eines Vibrationselements, eines optischen Elements oder Kombinationen davon aufweist.

6. System nach einem der Ansprüche 1 bis 5,
wobei der Vorgang des Berechnens der Differenz zwischen dem zweiten Datensatz und dem ersten Datensatz die Bestimmung aufweist, ob die Differenz zwischen dem zweiten Datensatz und dem ersten Datensatz statistisch signifikant ist.

7. System nach Anspruch 6,
wobei eine Methode der kleinsten Quadrate verwendet wird, um zu bestimmen, ob der Unterschied zwischen dem zweiten Datensatz und dem ersten Datensatz statistisch signifikant ist.

## Revendications

1. Système pour une utilisation en chirurgie réparatrice articulaire, le système comprenant :
- un dispositif de détection configuré pour une implantation à l'intérieur d'une articulation d'un patient, le dispositif de détection incluant au moins un capteur qui est configuré pour enregistrer au moins une force de réaction à l'intérieur de l'articulation tandis que l'articulation est déplacée à travers une plage de mouvements ;
- une pluralité d'implants d'essai configurés pour une implantation d'essai à l'intérieur de l'articulation du patient, chacun de la pluralité d'implants d'essai étant configuré pour simuler un implant d'une pluralité d'implants correspondants configurés pour une implantation permanente à l'intérieur de l'articulation ; et
- une mémoire non transitoire stockant électroniquement un code de programme exécutable par ordinateur, le code de programme exécutable par ordinateur étant configuré de telle sorte qu'un processeur, quand il exécute le code programme, devient un processeur à calcul spécifiquement programmé qui est configuré pour effectuer au moins les opérations suivantes :
- recevoir, depuis le dispositif de détection tandis que le dispositif de détection est positionné à l'intérieur d'une articulation d'un patient, un premier ensemble de données représentant une première série de forces de réaction à l'intérieur de l'articulation tandis que l'articulation est déplacée à travers une plage de mouvements durant une première période temporelle ;
- recevoir, depuis le dispositif de détection tandis que le dispositif de détection est positionné à l'intérieur de l'articulation du patient, un second ensemble de données représentant une seconde série de forces de réaction à l'intérieur de l'articulation tandis que l'articulation est déplacée à travers la plage de mouvements pendant une seconde période temporelle, dans lequel un premier implant parmi les implants d'essai est attaché à l'intérieur de l'articulation durant la première période temporelle ;
- calculer une différence entre le second ensemble de données et le premier ensemble de données ; et
- fournir une instruction sur la base de la différence, dans lequel l'instruction inclut soit (a) une instruction pour sélectionner un deuxième implant parmi les implants d'essai si la différence dépasse un seuil, soit (b) une instruction à effectuer avec un implant correspondant au premier implant parmi les implants d'essai si la différence ne dépasse pas le seuil,
- dans lequel la différence entre le second ensemble de données et le premier ensemble de données inclut une amplitude différente, et dans lequel le deuxième implant parmi les implants d'essai a une épaisseur différente du premier implant parmi les implants d'essai ; ou
- dans lequel la différence entre le premier ensemble de données et le second ensemble de données inclut un décalage de position, et dans lequel le deuxième implant parmi les implants d'essai a un décalage de position différent du premier implant parmi les implants d'essai,
**caractérisé en ce que**
- le dispositif de détection inclut un rail, et dans lequel ledit au moins un capteur de force peut être déplacé linéairement le long du rail.

2. Système selon la revendication 1,
comprenant en outre une pluralité d'implants réels, chacun de la pluralité d'implants réels correspondant à l'un de la pluralité d'implants d'essai, dans lequel la pluralité d'implants d'essai sont de préférence des implants d'essai rotuliens.

3. Système selon la revendication 1 ou 2,
dans lequel le seuil est soit un seuil absolu, soit un seuil relatif.

4. Système selon l'une des revendications 1 à 3,
dans lequel le dispositif de détection comprend une pluralité de capteurs de force, et
dans lequel la différence entre le second ensemble de données et le premier ensemble de données inclut une orientation différente, et dans lequel le second implant parmi les implants d'essai a un décalage angulaire différent du premier implant parmi les implants d'essai.

5. Système selon l'une des revendications 1 à 4,
dans lequel le dispositif de détection inclut au moins un élément parmi un élément résistif, un élément capacitif, un élément piézorésistif, un élément piézoélectrique, un élément hydraulique, un élément pneumatique, un élément magnéto-élastique, un élément vibratoire, un élément optique ou une combinaison de ceux-ci.

6. Système selon l'une des revendications 1 à 5,
dans lequel l'opération consistant à calculer la différence entre le second ensemble de données et le premier ensemble de données inclut de déterminer si la différence entre le second ensemble de données et le premier ensemble de données est statistiquement significative.

7. Système selon la revendication 6,
dans lequel la méthode des moindres carrés est utilisée pour déterminer si la différence entre le second ensemble de données et le premier ensemble de données est statistiquement significative.
